# EUROPEAN PATENT SPECIFICATION

(11) **EP 2 380 494 B1**
(45) Date of publication and mention of the grant of the patent: **27.01.2016**
(21) Application number: 11163628.8
(22) Date of filing: 26.04.2011
(51) Int. Cl.: A61B 5/11, A63B 24/00, A63B 71/06

(54) **Device, system and method for measurement of physical activity**
Vorrichtung, System und Verfahren zur Messung physischer Aktivität
Dispositif, système et procédé de mesure d'activité physique

(30) Priority: 15.06.2010 FI 20105689; 22.04.2010 FI 20100202 U
(43) Date of publication of application: 26.10.2011
(73) Proprietor: Modz Oy, 00810 Helsinki (FI)
(72) Inventor: Koski, Salla, 00990, Helsinki (FI); Varun, Singh, 00990, Helsinki (FI); Tasanen, Mikko, 00990, Helsinki (FI)
(74) Representative: IPR Partners Ltd

(56) References cited:
- WO-A2-02/067449
- US-A1- 2003 077 556
- US-A1- 2005 043 102
- US-A1- 2007 179 404
- US-A1- 2009 043 531
- US-B1- 7 052 472

## Description

### Field of invention

The invention relates to a device, system and method for measurement of physical activity. The invention is especially applicable for monitoring and inspiring physical exercise of e.g. children.

### Background technology

The number of over-weight people as well as people with poor physical condition has been increasing. This is much due to the change from physical to knowledge based work and hobbies. The problem of obesity has increased among all age groups; children, adolescents, adults and elderly people.

In order to ensure sufficient amount of physical activity it has become popular to monitor the activity with measurement devices. Activity can be measured in multiple methods. The simplest and earliest method to measure movement is a pedometer. This device measures the number of steps the user has taken and calculates the total distance based on the average length of the user's stride. The device is based on one-way bouncing mechanism. A more developed method to measure activity is a heart rate monitor. Although heart rate monitors don't actually measure movement, per se, an ascended heart rate is a result of either physical or mental stress, and thus reflects a person's activity and fit well. A third way for measuring activity is a GPS-receiver. This device is in contact with the Global Positioning System satellites and calculates a person's location based on a three-dimensional scale (latitude, longitude and altitude). This enables to track people's movement relatively accurately and also take into account the variations in altitude. A fourth technological innovation for measuring activity is a 3-dimensional accelerometer. This kind of accelerometers can detect the magnitude and direction of acceleration, which enables the measurement of various measures such as orientation, vibration and shock.

The activity measurement devices often have a possibility for data transfer with a personal computer or a server, whereby the user can get illustrations on the measurement results and make comparisons with personal activity targets. In network based applications it may also be possible to compare the results with other users of the service, and to have common targets for a group of users.

Patent application document US 2009/043531 A1 discloses a method for measuring acceleration of a human body with a portable device. The measurement is used for determining stride length. A device may also have means for providing wireless communication with various devices.

There are certain drawbacks in relation to the prior art devices described above. Adults may be interested in planning targets for one's activities and following the plans, but children and adolescents may not find it so interesting. Therefore, the prior art devices appear to lack an effect of inspiring the exercise activity, especially when children are concerned.

### Summary of the invention

The object of the invention is to provide a new solution for measuring and handling information which relates to physical exercise of a user, by which solution the above mentioned drawback can be decreased or avoided.

The object of the invention is achieved with a solution, in which a portable exercise measurement device of a user has means for transferring data with another measurement device and/or with a location indicating device and/or with other mobile device. The data transfer is preferably based on short range communication, such as Bluetooth, Near Field Communication (NFC), Radio Frequency Identification Device (RFID) technology, other 2,4 GHz radio link, usage of light such as infrared light, or usage of sound such as ultrasound.

The device according to the invention is well suited to different kinds of games including physical activity. For example, in one application of the invention the activity measurement device is used in a tag play. The devices are arranged to receive wireless signals from other measurement devices, and to check whether there is another device within a determined distance. At least one of the players is "tagged", and the tagged player is shown by a light of certain colour in the device of the tagged player, or "seeker". The seeker tries to catch other players, and when the seeker enters below the determined distance from another player the two measurement devices interact with each other, and the tagged player will change.

It is also possible that the device can be used with various other game applications. For example, the device may include GPS location measurement means, which may be used in a game. A user may load a map and a suggested route from a personal computer / network server, and the user may then move in the environment and try to follow the suggested route. The device may give feedback and instructions when the user is following the track. The actual route travelled by the user may then be loaded to a computer /server, whereby the game application may give further results, feedback and possible rewards/points.

In one preferable embodiment of the invention the device includes LED lights of different colours, and the lights are arranged to show the level of activity. For example, the current activity may be shown as a pulsed light, and the average activity may be shown with a continuous light. There may also be separate LED light(s) for physical game applications such as tag play. For example, LED light of a certain colour may indicate that the player is a seeker in the game. It is also possible to indicate the level of activity or a state in a physical game application by using sound as the user interface output or part of it.

It is also possible to transfer activity measurement results between measurement devices. In a physical game, the devices may receive activity results of other players, make a comparison, and to inform the level of activity compared to the other players. It is thus possible to compete with measured "activity points" within a group of players.

It is also possible to include additional devices in the system, the purpose of which is to indicate a determined location in a game. For example, it is possible to arrange orienteering wherein stationary devices are placed in control points. When a user finds a control point the measurement device interacts with the stationary device, and the measurement device gets an acknowledgement on visiting the control point. Of course, the location indicating devices may be a part of some other possible game instead of orienteering. For example, in a tag play the location indicating device may function as providing a safe are around the stationary device, wherein a player can stay for a moment without getting tagged.

The system may also be able to communicate with other mobile devices, such as a mobile phone, preferably a smart phone. With such a communication it is possible, for example, that parents receive data from the measurement device to their smart phone, and they are thus able to monitor the amount of exercise of their children. A measurement device may also connect to Internet services through via mobile station, via a pc or by connecting directly to a wireless network. The measurement device may use direct short range communication with a personal computer, a mobile station, or a game console, for example. However, it is also possible that the measurement device also has ability for such communications, WLAN or cellular communications, for example.

Further, it is possible to use the short range communication for transferring data to/from another measurement device used by the same user. For example, if a user needs a blood glucose meter, the meter may monitor the eating and physical exercising of the user. For this purpose the measurement device may transfer information for the glucose meter about the physical exercise of the user.

It is also possible that the short range communication is used for providing results of physical activity to a specific application of a smart phone, for example. The smart phone may thus show an avatar, for example, which will change in colour or other appearance on the basis of the result value. It is also possible that the measurement device may communicate with e.g. a cash terminal of a shop(such as a shop selling sports equipment), and the user may get bonus points for the shop on the basis of the physical exercise activities.

The system according to the present invention may also include a game program which is loaded on a server, for example, and to which program the users or a certain user group has an access. It is possible to set into the game program information on activity targets and measured activity results, for example. This way the users of the system can get activity points which they can use in games that are available with the server. Such a game may also be a social game or virtual world/community where a user may get virtual awards based on activity results, for example. Further, in one embodiment of the invention the system includes e.g. a chat application which is loaded on a server, into which program the users of the system have an access, whereby the users of the system can have contact with each other.

The inventive solution has significant advantages compared to the prior art. The interaction between the measurement devices makes it possible to provide live motional games between players, which inspires the physical activity. The players can be constantly aware of the situation of the play and/or activity points. It is not necessary to use a computer or a network service in order to find out the situation of the play / activity points within the play group. Thus it is possible to react to the changes in a play immediately, which inspires the participation in the game and activity. As a result, the physical activity can be increased without a need to have special interest in regular planning and monitoring of targets. The inventive solution is therefore very suitable for increasing the physical activity of users of all ages, including children and adolescents.

In one embodiment of the invention the system includes a game program which is loaded on a server, for example, to which program the users or a certain user group has an access via a communications network such as the Internet. It may be possible to set into the game program information of each user's targets and to transfer measurement results from each user's measurement device to the game program server. This way the users of the system can play with each other and, for example, get activity points or other benefits for the game on the basis of the measurement results. Further, in one embodiment of the invention the system includes e.g. a chat program which is loaded on a server, into which program the users of the system have an access, whereby the users of the system can have contact with each other.

In one aspect of the invention there is an attachment arrangement which allows the attachment of the measurement device or other portable device. The arrangement comprises an attachment part, whereby a formable material, such as a band or fabric of clothing can be fixed between the attachment part and the device housing. The device can thus be attached to the clothing of a user, or to the wrist, waist or neck of the user with a band.

A user portable device according to the invention for measurement of physical activity, is characterised by claim 1.

A method according to the invention, is characterised by claim 14.

Preferable embodiments of the invention are described in dependent claims

In this patent application "measurement device" means a device for measuring motional activity of a person directly with e.g. an acceleration or location detector, or indirectly with e.g. a heart rate monitor.

In this patent application "a user interface" means broadly means and programs relating to controlling a device by the user and/or providing information to the user, such as measurement related information, state information and interaction information.

A "user" of a measurement device means in this patent application a person, whose physical activity is measured with the measurement device.

In this patent application "short range communication" has a common meaning, and so the operating range may be less than 100 m, preferably less than 10 m and more preferably less than 1 m.

### List of drawings

In the following the invention is described with help of the enclosed drawings, in which:
- Figure 1: illustrates a block diagram of an exemplary measurement device according to the invention,
- Figure 2a: illustrates a top view of an exemplary measurement device according to the invention;
- Figure 2b: illustrates a perspective view of an exemplary arrangement for attachment of the measurement device.
- Figure 3: illustrates an example for presenting measurement data to a user with LED lights;
- Figure 4: illustrates an example of using a measurement device according to the invention in a tag play;
- Figure 5: illustrates a flow diagram of an exemplary use of a measurement device according to the invention in a tag play;
- Figure 6a: illustrates a block diagram of an exemplary arrangement according to the invention where measurement is used in a network; and
- Figure 6b: illustrates a block diagram of another exemplary system according to the invention where measurement is used in a network.

### Detailed description of exemplary embodiments

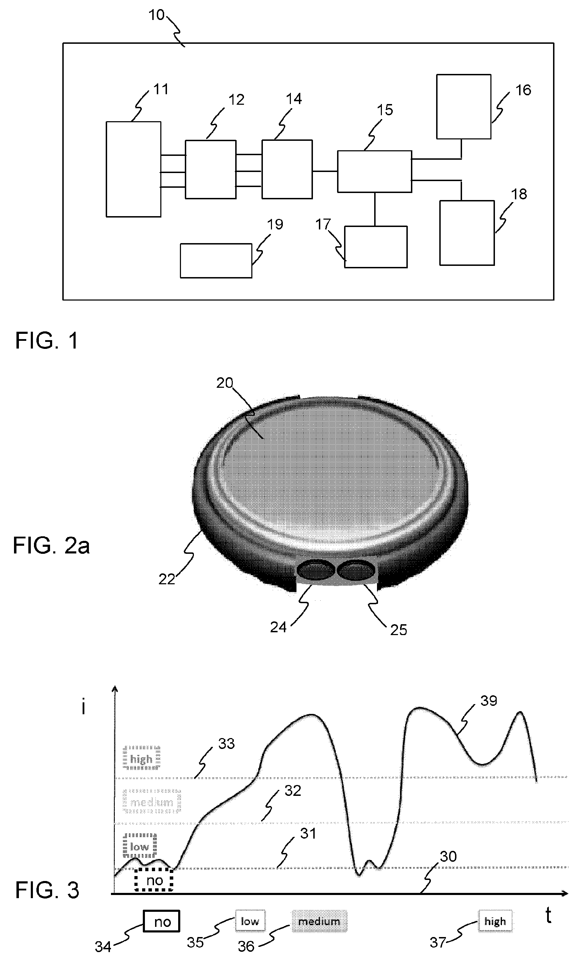

Figure 1 illustrates a block diagram of an exemplary device 10 according to the invention for measuring movement of the user. The device 10 has movement or acceleration sensors 11, the amount of which is preferably three. The movement sensors have been placed orthogonally related to each other for measuring movements in perpendicular directions. However, even if the use of acceleration sensors is considered preferable, it should be noted that the device may have alternatively another type of sensor, such as a heart rate sensor, a GPS receiver, or a pedometer. The device may also include a gyroscope for measuring changes in the orientation of the user. The gyroscope measurement may also include a 3-dimensional gyroscope measurement.

The signals received from the sensors are amplified in an amplifier 12 and converted into digital form with an A/D converter 14. The digital signals describing movement/acceleration are led to an input/inputs of a processor 15, from which the processor transfers the data into a memory 17 of the device. The processor saves the measurement results into a memory 17 for later use. Also the programs controlling the processor, and possible common and user specific parameters have been stored into the memory 17 of the measurement device. The processor may also calculate desired characteristic values from signals or it may filter the signals. Said A/D converter, amplifier and/or memory may also be included in the processor 15.

According to the invention the device can communicate with other measurement devices and/or with a location indicating device. The device may also communicate with a computer for loading programs/parameters to the measurement device or for transferring measurement data to the computer, server or other mobile device such as a mobile phone. The short range communication takes place by means of a transceiver part 16 wirelessly with e.g. radio data transfer of Bluetooth type. Other communication possibilities may be NFC or RFID technology, or a 2,4 GHz link, for example. The transceiver may also be integrated in the processor. Data transfer with a computer may also be based on wired connection such as e.g. USB interface.

The user interface means 18 of the device may include LED lights, a LCD, LED or OLED matrix display, and/or a sound forming means in order to provide information for the user. Signals for light control and possible audio signals corresponding to tones or speech can be formed in the processor by means of data, such as a program and parameters, stored in the memory. The user interface may also include e.g. push button switches for controlling the device.

The device of Figure 1 also includes an energy source 19, such as a rechargeable or disposable battery.

A location indicating device may be a measurement device which has been set into a location indicating state with a specific press button switch, for example. It is also possible that the measurement devices of the players are identified at the beginning of the play, and the not identified devices are treated as location indicating devices. It is also possible to provide a specific location indicating device, which does not include an activity measurement sensor and related circuits.

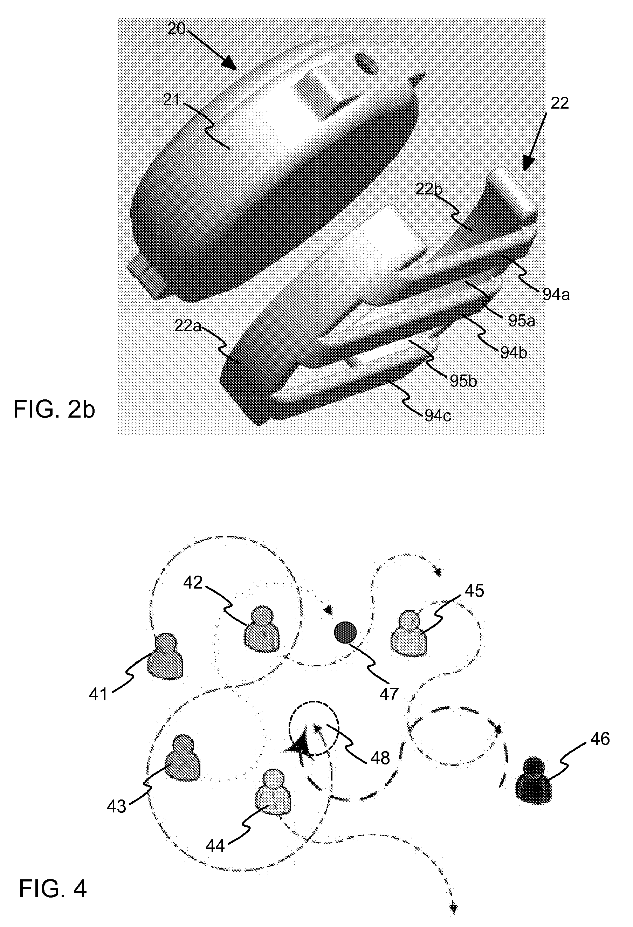

Figures 2a and 2b illustrate an exemplary device 20 according to the invention for measuring physical activities of a user. The device has two push button switches 24 and 25, wherein one of the switches may serve as on/of switch for the device. The other switch may be used for specific applications, such as a physical game application. If used with a tag game, for example, the game can be started by pressing the button. The player can also manually change its role as a seeker or runner by pressing the button switch. The game can be stopped by turning off the device, for example. The switches are not shown in Figure 2b; the planar area at the side of the device housing is reserved for the switches. It is naturally also possible to use other than push button switches, such as a toggle switch, for example.

The device of Figure 2a has an attachment part 22 for attaching the device. The attachment part 22 is illustrated in more detail in Figure 2b. The housing of the measurement device has a round side 21. The walls 22a and 22b of the attachment part have a corresponding arched form, thus following the form of the device housing. The walls 22a and 22b of the attachment part are fixed to each other with three supports 94a, 94b ja 94c. There are two apertures 95a and 95b between the supports for threading a possible band through the apertures. Such a band can be used for attaching the device (attachment part) to the wrist, waist or neck of a user. Alternatively it is possible to attach the device to a user's clothing by pressing the cloth fabric between the attachment part and the device housing. The device is preferably kept in a fixed orientation in relation to the user so that the signals from the sensors of the device describe desired directions of movement.

When the measurement device is attached to a formable material, such as fabric of clothing, the material is placed between the device housing 20/21 and the attachment part 22. If the formable material is a band, it can be threaded through the apertures 95a and 95b so that the band circulates around the support 94b at the opposite side of the device. The band circulates around the supports 94a and 94c between the device housing and the attachment part, The band will thus remain attached between the device and the attachment part when the device is placed in the attachment part. The walls 22a, 22b of the attachment part are then squeezed against the outer walls 21 of the device housing thus fixing the device. On the other hand, if the formable material is a larger-sized cloth fabric, the fabric will be located between the device housing and the attachment part also at the side walls of the device/attachment part. In this case the squeezing force of the walls of the attachment part as well as the friction between the materials will keep the device housing, attachment part and the fabric between them attached to each other. The device will thus be fixed to the formable material, such as a cloth of the user. Such a cloth may be a shirt or a scarf, for example.

The distance between the attachment part walls 22a, 22b as well as the rigidity of the supports 94a, 94b ja 94c is dimensioned such that the walls are squeezed against the sides of the device housing with a suitable force independently on whether there is a formable material between the device housing and the attachment part or not. The outer surface of the device housing sides and the inner surfaces of the attachment part walls are preferably rough in order to increase the attachment friction. As a result, the device and the attachment part are manually attachable and releasable to/from each other, but they are not released inadvertently.

It should be noted that the above mentioned feature concerning the attachment of a mobile device can also be used independently of the other features described in this patent application. Other applications may include, for example, attachment of auxiliary devices for autistic individuals or individuals suffering paramnesia.

The device of Figure 2a has LED lights for providing information for the user. The LEDs are located under the translucent front cover, and there are LEDs of different colours for different meanings. The LEDs may be bright enough to illuminate the whole translucent cover. The information provided by LED lights is easy to understand, especially for child users. However, there are naturally many other alternative possibilities for providing the information to the user, such as an LCD display, OLED display or sound providing means.

In one alternative embodiment, the device has a first user interface at its first surface, and a second user interface at its second surface. It is then possible to attach the device to the attachment part in a first position where the first surface becomes a front surface, or to a second position where the second surface becomes the front surface. In the first position, the user interface of the first surface is used, and in the second position, the second user interface is used. For example, if a device has two operational modes, such as measuring physical activity of the user and a tag game application, there may be user interfaces for both operational modes, at the opposite surfaces of the device. The device is thus attached to the attachment part in such a position that the operational mode which is currently used will be at the front.

If a physical activity measurement is selected as the operational mode, for example, the user interface at the corresponding first surface may include LED lights and/or sound providing means for displaying the momentary or cumulative level of physical activity. The first user interface may also include a press button switch for switching the device (and the physical activity measurement mode) on/off. The press button switch may also be used for selecting the display mode between a momentary and cumulative level display, whereby pressing the button for a short period will change the display mode. Pressing the button for a longer period, on the other hand, may switch the device off.

If a tag game application is selected as the operational mode, for example, the user interface at the corresponding second surface may include LED lights and/or sound providing means for displaying the state of the player. The second user interface may also include a press button switch for switching the device (and the tag game mode) on/off. The press button switch may also be used for selecting the state of the player (e.g. runner/seeker), whereby pressing the button for a short period will change the state. Pressing the button for a longer period, on the other hand, may switch the device off.

It should be noted that the above mentioned feature can also be used independently in other types of devices, especially portable devices, than physical activity measurement devices or game applications. The other applications may include functionalities of a mobile phone, a music player, a radio or a clock, for example. The general feature may thus be defined as having two operational modes in a device, and two user interfaces at the opposite surfaces of the device for using the device in the corresponding two operational modes, and preferably including a possibility to select the position of the device from two positions, wherein in the first position the first surface including at least part of the first user interface is the front surface, and in the second position the second surface including at least part of the second user interface is the front surface of the device. The device is preferably attached to an attachment part, wherein the device can be attached to in the two alternative positions. The attachement part may preferably be further attached to clothing of a user, or to a strap for the attachment to the user's wrist, waist or neck.

Figure 3 illustrates a graphical presentation of exemplary measurement data: the graph 30 shows the movement intensity i as a function of time t. The measurement device displays the information on the physical activity with LEDs of four colours; white, red, yellow and green. Momentary activity is displayed with a pulsed light, and cumulative activity is displayed with a continuous light. If momentary and cumulative activity are displayed with a same colour, the LED shows a pulsed light changing between two levels of light of the concerned colour. According to another possibility, the LEDs show either momentary or cumulative activity. It is possible to select between these activity display modes by pressing a press button switch of the device, for example. A same press button switch of the device may serve e.g. as an on/off switch and activity display mode selection switch.

In the graph of Figure 3, there are three predetermined activity threshold levels 30, 31, 32 and 33. Activity level 30 means zero or close to zero activity. When the measured momentary activity level is between threshold values 30 and 31, the white LED is blinking. When the measured momentary activity level is between threshold values 31 and 32, the red LED is blinking. When the measured momentary activity level is between threshold values 32 and 33, the yellow LED is blinking. When the measured momentary activity level is above threshold value 33, the green LED is blinking.

There are also predetermined threshold values for cumulative activity level. At the beginning of the graph, the measured cumulated activity value is threshold values 30 and 31, white LED is lit continuously, 34. When the measured cumulated activity value exceeds the threshold level 31, red LED is continuously lit, 35. When the measured cumulated activity value exceeds the threshold level 32, yellow LED is continuously lit, 36. When the measured cumulated activity value exceeds the threshold level 33, green LED is continuously lit, 37. This way the device informs the user on measured momentary activity as well as cumulative activity. The cumulative activity may be, for example, achieved by integrating the momentary activity values measured during a day, and the momentary activity may be, for example, achieved by integrating the momentary activity values measured during one second.

The acceleration signals received from the sensors can be used as such to indicate activity. However, after integrating the signals, velocity information is achieved, which can also be used to indicate physical activity level. It is also possible that the processor calculates an activity index by weighting different components of movement with suitable weights and calculating an average value of the components, for example.

Figure 4 illustrates an example of using a measurement device according to the invention in a tag play. There are six players 41-46 in the game, each player equipped with an activity measurement device. It is shown with dotted lines how each of the players will move. At the starting position the player 46 is seeker, and other players are runners, trying to avoid getting caught by the seeker. The measurement device of the seeker in a seeker state, which is shown by a LED light of determined colour in the measurement device of the seeker. The other players may have a LED light with a colour meaning a runner in their measurement devices. At the end position the seeker 46 catches the runner 41 in location 48. The measurement devices of the players 41 and 46 detect that the distance between the measurement devices is shorter than a determined threshold value. In this situation the measurement devices transfer their status information to each other. When the status of the devices is different, the devices both change their status. The player 41 thus becomes a seeker and the player 46 becomes a runner.

It is also possible that the game includes a location indicating device 47. Such a device may be installed in a suitable location in the game environment. The location indicating device does not measure movement but it only has a function of communicating with the measurement devices when they are within the operating range. It is possible, for example, an area near to the location indicating device is a safe area for runners, i.e. a runner can stay in that area for a predetermined time period without getting caught. When a measurement device detects the vicinity of a location indicating device, it may thus disable the change of its state for a predetermined period, such as 15 seconds. However, the area around a location indicating device may naturally have various possible functions in a game.

The measurement device may naturally also provide information on the physical activity of the user during a game, as was described in relation to Figure 3, for example.

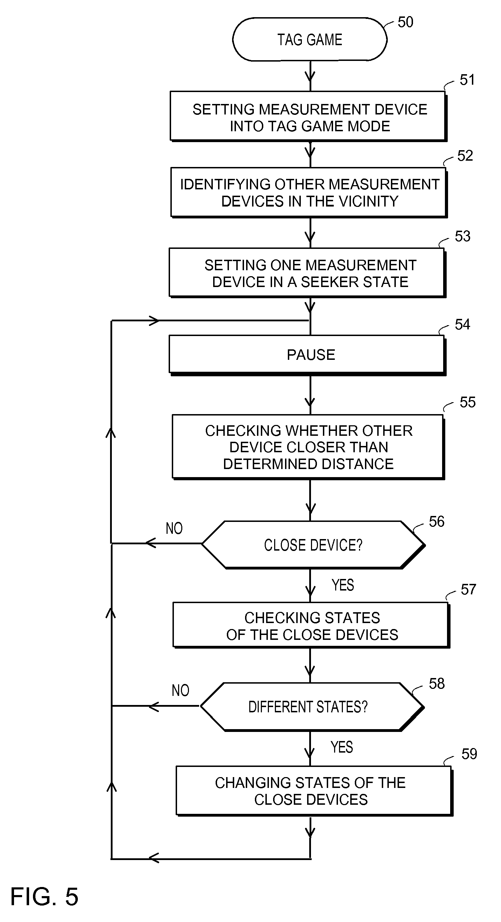

Figure 5 illustrates a flow diagram of an exemplary method 50 where measurement devises are used in a tag play. The measurement devices are first set into tag game mode in phase 51. Than can be made by e.g. pressing a game button of each device. In the next phase 52 the devices use their short range communication in order to identify other nearby devices, and to form a player group including the identified devices. In this phase the devices of the players must be close enough to facilitate the short range communication. Phase 52 is optional, but it may be useful that the devices of the players are identified in order to avoid any outside devices interacting with the game.

In phase 53 one measurement device is set into seeker state. This can be done, for example, by pressing the game button again. The measurement device may have a functionality in which it changes its game state each time when press button is pressed. After changing a game state of a measurement device it is preferable to include a pause phase 54, in the method, which may be e.g. 15 seconds. This way it is possible avoid successive, continuous change of states between nearby devices.

After the seeker has been selected and activated, the game actually starts. In phase 55 the seeker device is checking whether there are other devices at a close distance to the seeker device. There may be a predetermined threshold value for the distance, whereby a device is defined as "close" when the distance between the devices is lower than the threshold value. The distance between the devices is thus measured in some manner. The threshold distance may be the operating distance of the short range communication. In this way, all devices that can communicate with each other using the short range communication are in a close distance with each other. It is also possible to measure the strength of a signal transmitted from another device, and to determine the distance on the basis of the received signal strength. If the devices include a GPS module, the location information received from this module may also be used for determining the distance between two devices after the location information has been transferred between the devices. However, each of these methods require the short range communication for determining the distance.

If there is no other device within the determined distance, 58, the checking 55 is repeated after a pause 54. When there is another device located closer to the seeker device than the determined threshold distance, the devices may check each other's states, phase 57. Checking the states can be optional, if only the seeker checks other devices, and if only one seeker is allowed in the game. If the states of the close devices are different, 58, the states of the close devices are changed in phase 59. The seeker thus gets a runner state and the runner gets a seeker state. After a pause 54, the checking of close devices continues by the new seeker. If the states appear the same, 58, the states are not changed, but the checking 55 will continue after a pause 54. With situation may appear if checking 55 is made by all devices, or if two or several seekers are allowed in the game. During the game, it is also preferable that the activity levels of a user are shown as was described in relation to Figure 3, for example.

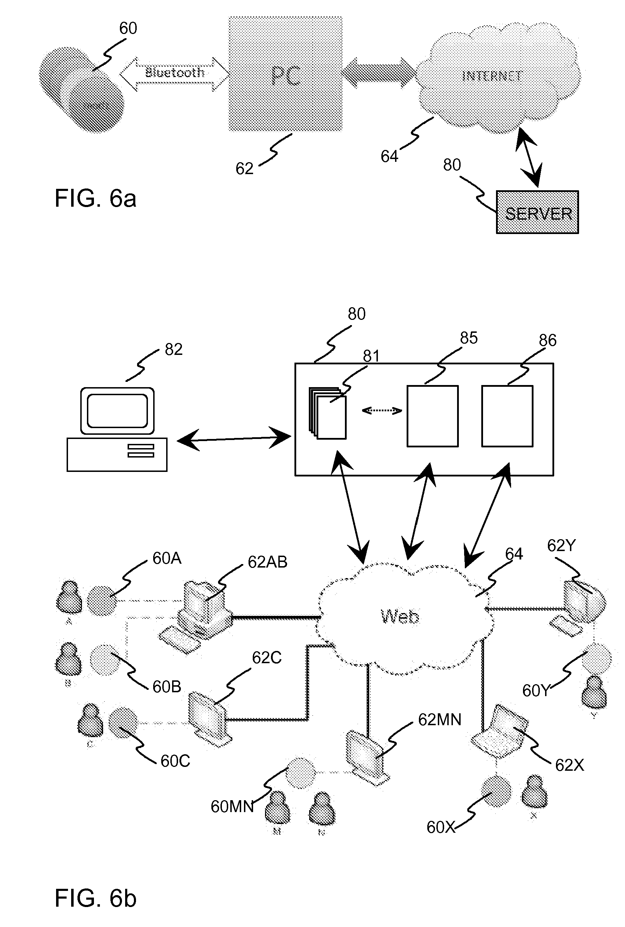

Figure 6a illustrates a block diagram of an exemplary arrangement for transferring data between a measurement device 60, personal computer 62 and a network server 60. The measurement device may be preferably connected to a personal computer with a same short range communication functionality, such as Bluetooth, which is used for communication with other measurement devices. However, it is also possible to use wired connection between the measurement device and a personal computer, such as an USB connection. The connection can be used for transferring programs and parameters into the measurement device, and for transferring measurement results or related activity points to the personal computer from the measurement device. The personal computer may include software for displaying measurement results in a graphical manner, for example. The personal computer may further be connected to a server 80 through Internet 64, for example. The server may include game applications, for example, wherein measurement results or activity points may be used

Figure 6b illustrates a system with several measurement devices and users. Users A, B, C, X and Y each have a measurement device for their own personal use. However, it is also possible that one measurement device has two or several occasional users, like users M and N. Measurement devices 60C, 60MN, 60X and 60Y are connected to personal computers 62C, 62MN, 62X and 62Y, respectively. However, players A and B use a common personal computer for the connection with their measurement devices 60A and 60B. In cases where several users use a same measurement device or a same personal computer, the identity of the actual user is input to the computer in connection with authentication for each use, and thus the user data can be kept separate for each user. Although a personal computer is mentioned here as a terminal, it is naturally possible that other alternative terminal type is used, such as a mobile communicator.

The system according to Figure 6b also includes a server 80, which may have game applications 85 in which e.g. child users of the system can play with each other. Playing a game takes place with the terminal equipment 62AB...62Y by transferring game related data between the terminal equipment and the server application 85 via the data transfer network 64. The program stored in the server 80 functions in such a way, for example, that the measurement results / activity points of the users are stored, and it is possible to use activity points in the game as valuables, or it may even be possible to win actual prizes. It may also be possible that activity points are required for getting a right to play a game for a certain period. The program includes preferably an identification functionality, by which the users log in to use the game. The users can appear in the game with their own name or with a pseudonym, for example. The right of use of the game can be preferably arranged in such a way that a such user can get a user right to the game who is provided with a measurement devices and/or software of a terminal equipment of a certain user group. The server 80 comprises a database 81, into which personal information and information relating to the measurement devices and their users is stored. A system administrator has an access to the central computer via e.g. a terminal equipment 82, after logging in to the system.

The data transfer network 64 can be e.g. a wired Internet or a wireless data transfer network of a cellular communication system. It must be noted that the data transfer means of the measurement device can alternatively connect directly to the data transfer network 64 in such a way that e.g. the transfer of the measurement data / activity points to the server 80 can take place without a separate terminal equipment. In such a case the data transfer means can be e.g. a GSM or 3G cellular communication module, into which a SIM card of the user may be connected.

Above, some systems and devices according to the invention have been described. The functionality according to the invention is achieved with, in addition to the devices mentioned herein, by storing the programs which relate to the inventive functions and which control the processor/processors into the memories of the devices of the system. Programming measurement devices is known as such for a person skilled in the art, and he/she can implement the functions of the present invention on the basis of the description given here.

It must be noted that above only some embodiments of the solution according to the invention have been described. The principle of the invention can naturally be modified within the scope of protection determined by the patent claims, e.g. in details of implementation and areas of use.

The present invention is not, for example, restricted to user interface means or data transfer types or functions which were described in the above embodiments, but a person skilled in the art can design several alternative implementations in the frame of the inventive features.

The present invention is also not restricted to devices which only have functionalities that relate to of physical activities. It is possible, for example, that the features of the present invention are integrated in a mobile station or some other device, which can then be used for inspiring physical activities of a user in accordance with the present invention.

It should also be noted that the feature of providing wireless short range communication with a second device, and the device being arranged to provide information for the user, which information is at least in part based on said short range communication, together with other related features, may also be applied in other devices than devices for the measurement of physical activity of a user. For example, it is possible that a device is provided for a tag game, wherein there are provided functionalities on short range communication and distance measurement between two corresponding devices and/or with a location indicating device, but the tag game device does not include the means for the measurement of the level of physical activity of the user. The features relating to the short range communication / distance measurement of portable devices may also be applied in e.g. game applications independently of the measurement of physical activity.

## Claims

1. A user portable device (20) configured to measure physical activity, which comprises measurement means (11) configured to measure physical activity of the user, and a user interface (18) configured to present to a user information which is based on a measurement result, wherein the device comprises means (16) configured to provide wireless short range communication with a second device, and the device is arranged to provide information for the user, which information is at least in part based on said short range communication, **characterised in that** the device comprises two operational modes, and two user interfaces at the opposite surfaces of the device configured to use the device in the corresponding two operational modes.

2. The measurement device (20) according to claim 1, **characterised in that** the second device is a portable device for measurement of physical activity of another user.

3. The measurement device (20) according to claim 1 or 2, **characterised in that** the second device is a location indicating device.

4. The measurement device (20) according to any preceding claim, **characterised in that** the wireless short range communication is based on radio signal, induction, light or sound.

5. The measurement device (20) according to any preceding claim, **characterised in that** the short range communication is based on Bluetooth, Near Field Communication (NFC) or Radio Frequency Identification (RFID).

6. The measurement device (20) according to any preceding claim, **characterised in that** it has means for indicating the distance to said second measurement device or said location indicating device, whereby the information provided to the user is at least in part based on said indication of the distance.

7. The measurement device (20) according to any preceding claim, **characterised in that** it has a game application with a logical state, wherein the logical state is at least in part based on the short range communications with the second device.

8. The measurement device (20) according to any preceding claim, **characterised in that** the device has user interface means for providing information to the user, whereby the user interface means (18) comprise light forming means, such as LEDs of different lights, a display, and/or sound providing means.

9. The measurement device (20) according to any preceding claim, **characterised in that** said device includes a possibility to select the position of the device from two positions, wherein in the first position the first surface including at least part of the first user interface is the front surface, and in the second position the second surface including at least part of the second user interface is the front surface of the device.

10. The measurement device (20) according to any preceding claims, **characterised in that** the first operational mode of said device is measuring physical activity of the user and the second operation mode is a tag game application.

11. The measurement device (20) according to any preceding claim, **characterised in that** it comprises an attachment arrangement, wherein the attachment arrangement has an attachment part (22) which is attachable to the attachment between the attachment part and a housing of the measurement device based on friction, the device housing and the attachment part being manually attachable and releasable to/from each other and whereby the measurement device is attachable to a formable material by placing at least part of the formable material between the housing of the measurement device and the attachment part.

12. The measurement device (20) according to any preceding claim, **characterised in that** the measurement device is part of a system for measuring at least one magnitude which relates to physical activity of user, which system comprises several measurement devices and optionally a location indicating device, wherein the measurement devices comprise means for short range communication with other devices of the system, such as other measurement devices of the system and/or an optional location indicating device, and the measurement devices are arranged to provide information to the user, which information is at least in part based on said communication.

13. The measurement device (20) according to claim 12, **characterised in that** the system comprises a game application in a network server and means for storing information relating to a measurement results, means for arranging access for a user of the system into the game program and an arrangement for using said information in the game program.

14. A method in which physical activity of a user is measured and the measurement result is presented to a user with a user portable measurement device, wherein short range communication is provided between the measurement device and a second device, and information is provided for the user by the measurement device, which information is at least in part based on said short range communication, **characterised in that** the method is implemented with the measurement device (20) according to any of the claims 1-13.

15. The method according to claim 14, **characterised in that** a game application is run in the measurement device, and a logical state of the game application is changed based on said short range communication between the devices.

## Patentansprüche

1. Von einem Benutzer tragbares Gerät (20), das konfiguriert ist, um physische Aktivität zu messen, das Messmittel (11), die konfiguriert sind, um physische Aktivität des Benutzers zu messen, und eine Benutzerschnittstelle (18) aufweist, die konfiguriert ist, um einem Benutzer Informationen zu präsentieren, die auf einem Messergebnis basiert, wobei das Gerät Mittel (16) aufweist, die konfiguriert sind, um drahtlose Nahbereichskommunikation mit einem zweiten Gerät bereitzustellen, und das Gerät gestaltet ist, um Information für den Benutzer bereitzustellen, wobei die Information zumindest teilweise auf der Nahbereichskommunikation basiert, **dadurch gekennzeichnet, dass** das Gerät zwei Betriebsmodi und zwei Benutzerschnittstellen auf den gegenüberliegenden Seiten des Geräts aufweist, die konfiguriert sind, um das Gerät in den entsprechenden zwei Betriebsmodi zu verwenden.

2. Messgerät (20) nach Anspruch 1, **dadurch gekennzeichnet, dass** das zweite Gerät ein tragbares Gerät zur Messung von physischer Aktivität eines weiteren Benutzers ist.

3. Messgerät (20) nach Anspruch 1 oder 2, **dadurch gekennzeichnet, dass** das zweite Gerät ein Ortsanzeigegerät ist.

4. Messgerät (20) nach einem vorangehenden Anspruch, **dadurch gekennzeichnet, dass** die drahtlose Nahbereichskommunikation auf einem Funksignal, Induktion, Licht oder Schall basiert.

5. Messgerät (20) nach einem vorangehenden Anspruch, **dadurch gekennzeichnet, dass** die Nahbereichskommunikation auf Bluetooth, Nahfeldkommunikation (Near Field Communication (NFC)) oder Funkfrequenzkennzeichnung (Radio Frequency Identification (RFID)) basiert.

6. Messgerät (20) nach einem vorangehenden Anspruch, **dadurch gekennzeichnet, dass** es Mittel zum Anzeigen der Entfernung zum zweiten Messgerät oder zum Ortsanzeigegerät aufweist, wobei die dem Benutzer bereitgestellte Information zumindest teilweise auf der Anzeige der Entfernung basiert.

7. Messgerät (20) nach einem vorangehenden Anspruch, **dadurch gekennzeichnet, dass** es eine Spieleanwendung mit einem logischen Zustand aufweist, wobei der logische Zustand zumindest teilweise auf den Nahbereichskommunikationen mit dem zweiten Gerät basiert.

8. Messgerät (20) nach einem vorangehenden Anspruch, **dadurch gekennzeichnet, dass** das Gerät Benutzerschnittstellenmittel zum Bereitstellen von Information für den Benutzer aufweist, wobei die Benutzerschnittstellenmittel (18) Lichtformmittel, wie z. B. LEDs mit verschiedenen Lichtern, eine Anzeigeeinrichtung und/oder Tonbereitstellungsmittel aufweisen.

9. Messgerät (20) nach einem vorangehenden Anspruch, **dadurch gekennzeichnet, dass** das Gerät eine Möglichkeit zum Auswählen der Position des Geräts aus zwei Positionen enthält, wobei in der ersten Position die zumindest einen Teil der ersten Benutzerschnittstelle enthaltende erste Seite die Vorderseite ist und in der zweiten Position die zumindest einen Teil der zweiten Benutzerschnittstelle enthaltende zweite Seite die Vorderseite des Geräts ist.

10. Messgerät (20) nach einem vorangehenden Anspruch, **dadurch gekennzeichnet, dass** der erste Betriebsmodus des Geräts ein Messen von physischer Aktivität des Benutzers ist und der zweite Betriebsmodus eine Fangenspielanwendung ist.

11. Messgerät (20) nach einem vorangehenden Anspruch, **dadurch gekennzeichnet, dass** es eine Befestigungsanordnung aufweist, wobei die Befestigungsanordnung einen Befestigungsteil (22) aufweist, der an der Befestigung zwischen dem Befestigungsteil und einem Gehäuse des Messgeräts basierend auf Reibung befestigbar ist, wobei das Gerätegehäuse und der Befestigungsteil von Hand aneinander befestigbar und voneinander lösbar sind, und wodurch das Messgerät an einem formbaren Material durch Platzieren von zumindest einen Teil des verformbaren Materials zwischen dem Gehäuse des Messgeräts und dem Befestigungsteil befestigbar ist.

12. Messgerät (20) einem vorangehenden Anspruch, **dadurch gekennzeichnet, dass** das Messgerät Teil eines Systems zum Messen von zumindest einer Größe ist, die sich auf die physische Aktivität eines Benutzers bezieht, wobei das System mehrere Messgeräte und optional ein Ortsanzeigegerät aufweist, wobei die Messgeräte Mittel zur Nahbereichskommunikation mit anderen Geräten des Systems, wie z. B. anderen Messgeräten und/oder einem optionalem Ortsanzeigegerät, aufweisen und die Messgeräte gestaltet sind, um Information für den Benutzer bereitzustellen, wobei die Information zumindest teilweise auf der Kommunikation basiert.

13. Messgerät (20) nach Anspruch 12, **dadurch gekennzeichnet, dass** das System eine Spieleanwendung in einem Netzwerkserver und Mittel zum Speichern von Information bezüglich eines Messergebnisses, Mittel zum Erreichen eines Zugangs für einen Benutzer des Systems in das Spieleprogramm und eine Anordnung zur Verwendung der Information in dem Spieleprogramm aufweist.

14. Verfahren, bei dem physische Aktivität eines Benutzers gemessen und das Messergebnis einem Benutzer mit einem von einem Benutzer tragbaren Messgerät präsentiert wird, wobei Nahbereichskommunikation zwischen dem Messgerät und einem zweiten Gerät bereitgestellt wird und Information für den Benutzer durch das Messgerät bereitgestellt wird, wobei die Information zumindest teilweise auf der Nahbereichskommunikation basiert, **dadurch gekennzeichnet, dass** das Verfahren mit dem Messgerät (20) nach einem der Ansprüche 1 bis 13 implementiert ist.

15. Verfahren nach Anspruch 14, **dadurch gekennzeichnet, dass** eine Spieleanwendung in dem Messgerät läuft und ein logischer Zustand der Spieleanwendung basierend auf der Nahbereichskommunikation zwischen den Geräten geändert wird.

## Revendications

1. Dispositif portable d'utilisateur (20) configuré pour mesurer l'activité physique, qui comprend des moyens de mesure (11) configurés pour mesurer l'activité physique de l'utilisateur, et une interface utilisateur (18) configurée pour présenter à un utilisateur des informations qui reposent sur un résultat de mesure, dans lequel le dispositif comprend des moyens (16) configurés pour assurer une communication sans fil à courte portée avec un second dispositif, et le dispositif est agencé pour fournir des informations pour l'utilisateur, lesdites informations reposant au moins en partie sur ladite communication à courte portée, **caractérisé en ce que** le dispositif comprend deux modes opérationnels, et deux interfaces utilisateur au niveau des surfaces opposées du dispositif configurées pour utiliser le dispositif dans les deux modes opérationnels correspondants.

2. Dispositif de mesure (20) selon la revendication 1, **caractérisé en ce que** le second dispositif est un dispositif portable pour la mesure de l'activité physique d'un autre utilisateur.

3. Dispositif de mesure (20) selon la revendication 1 ou 2, **caractérisé en ce que** le second dispositif est un dispositif d'indication d'emplacement.

4. Dispositif de mesure (20) selon l'une quelconque des revendications précédentes, **caractérisé en ce que** la communication sans fil à courte portée repose sur un signal radio, une induction, une lumière ou un son.

5. Dispositif de mesure (20) selon l'une quelconque des revendications précédentes, **caractérisé en ce que** la communication à courte portée repose sur la technologie Bluetooth, la communication en champ proche (CCP) ou l'identification par radiofréquence (RFID).

6. Dispositif de mesure (20) selon l'une quelconque des revendications précédentes, **caractérisé en ce qu'**il comprend des moyens pour indiquer la distance par rapport audit second dispositif de mesure ou audit dispositif d'indication d'emplacement, moyennant quoi les informations fournies à l'utilisateur reposent au moins en partie sur ladite indication de la distance.

7. Dispositif de mesure (20) selon l'une quelconque des revendications précédentes, **caractérisé en ce qu'**il comprend une application de jeu comprenant un état de logique, dans lequel l'état de logique repose au moins en partie sur les communications à courte portée avec le second dispositif

8. Dispositif de mesure (20) selon l'une quelconque des revendications précédentes, **caractérisé en ce que** le dispositif comprend des moyens d'interface utilisateur pour fournir des informations à l'utilisateur, moyennant quoi les moyens d'interface utilisateur (18) comprennent des moyens de formation de lumière, tels que des DEL de différentes lumières, une unité d'affichage et/ou des moyens de fourniture de son.

9. Dispositif de mesure (20) selon l'une quelconque des revendications précédentes, **caractérisé en ce que** ledit dispositif comprend une possibilité de sélectionner la position du dispositif à partir de deux positions, dans lequel, dans la première position, la première surface comprenant au moins une partie de la première interface utilisateur est la surface avant, et dans la seconde position, la seconde surface comprenant au moins une partie de la seconde interface utilisateur est la surface avant du dispositif.

10. Dispositif de mesure (20) selon l'une quelconque des revendications précédentes, **caractérisé en ce que** le premier mode opérationnel dudit dispositif mesure l'activité physique de l'utilisateur et le second mode opérationnel est une application de jeu de poursuite.

11. Dispositif de mesure (20) selon l'une quelconque des revendications précédentes, **caractérisé en ce qu'**il comprend un système de fixation, dans lequel le système de fixation comprend une partie de fixation (22) qui peut être fixée à la fixation entre la partie de fixation et un boîtier du dispositif de mesure reposant sur un frottement, le boîtier du dispositif et la partie de fixation pouvant être manuellement fixés l'un à l'autre et libérés l'un de l'autre et moyennant quoi le dispositif de mesure peut être fixé à un matériau façonnable en plaçant au moins une partie du matériau façonnable entre le boîtier du dispositif de mesure et la partie de fixation.

12. Dispositif de mesure (20) selon l'une quelconque des revendications précédentes, **caractérisé en ce que** le dispositif de mesure fait partie d'un système pour mesurer au moins une grandeur associée à l'activité physique d'un utilisateur, ledit système comprenant plusieurs dispositifs de mesure et éventuellement un dispositif d'indication d'emplacement, dans lequel les dispositifs de mesure comprennent des moyens pour une communication à courte portée avec d'autres dispositifs du système, tels que d'autres dispositifs de mesure du système et/ou un éventuel dispositif d'indication d'emplacement, et les dispositifs de mesure sont agencés pour fournir des informations à l'utilisateur, lesdites informations reposant au moins en partie sur ladite communication.

13. Dispositif de mesure (20) selon la revendication 12, **caractérisé en ce que** le système comprend une application de jeu dans un serveur de réseau et des moyens pour stocker des informations associées à des résultats de mesure, des moyens de fourniture d'accès pour un utilisateur du système au programme de jeu et un système pour utiliser lesdites informations dans le programme de jeu.

14. Procédé dans lequel l'activité physique d'un utilisateur est mesurée et le résultat de mesure est présenté à un utilisateur grâce au dispositif de mesure portable d'utilisateur, dans lequel une communication à courte portée est assurée entre le dispositif de mesure et un second dispositif, et des informations sont fournies pour l'utilisateur par le dispositif de mesure, lesdites informations reposant au moins en partie sur ladite communication à courte portée, **caractérisé en ce que** le procédé est mis en oeuvre grâce au dispositif de mesure (20) selon l'une quelconque des revendications 1 à 13.

15. Procédé selon la revendication 14, **caractérisé en ce qu'**une application de jeu est exécutée dans le dispositif de mesure, et un état de logique de l'application de jeu est modifié sur la base de ladite communication à courte portée entre les dispositifs.
